# EUROPEAN PATENT APPLICATION

(11) **EP 4 324 430 A1**
(43) Date of publication of application: **21.02.2024**
(21) Application number: 22191063.1
(22) Date of filing: 18.08.2022
(51) Int. Cl.: A61F 2/28, A61B 34/10, A61F 2/30

(54) **METHOD FOR PRODUCING A BONE REPLACEMENT ELEMENT**

(71) Applicant: Fraunhofer-Gesellschaft zur Förderung der angewandten Forschung e.V., 80686 München (DE); Albert-Ludwigs-Universität Freiburg, 79098 Freiburg (DE)
(72) Inventor: GUTMANN, Florian, 79115 Freiburg (DE); HOSCHKE, Klaus, 79102 Freiburg (DE); NELSON, Katja, 79104 Freiburg (DE); ROTHWEILER, René, 79114 Freiburg (DE); GROSS, Christian, 79098 Freiburg (DE)
(74) Representative: Pfenning, Meinig & Partner mbB

(57) **Abstract**

The invention relates to a method for producing a bone replacement element, wherein an image of a defect to be filled with the bone replacement element is acquired (S1), and at least one region with a specific architecture is defined within the outer contour (S3) of the defect (S3). A model is constructed for each of the regions (S4), wherein the model is defined by at least one topological parameter. Each model is constructed by defining a start model which is defined by at least one start value of the at least one topological parameter and the topological parameters are then fitted such that the model matches anatomical parameters within a predefined accuracy. The bone replacement element is then produced according to the model so obtained (S5).

## Description

The invention relates to a method for producing a bone replacement element, wherein an image of a defect to be filled with the bone replacement element is acquired, and at least one region with a specific architecture is defined within the outer contour of the defect. A model is constructed for each of the regions, wherein the model is defined by at least one topological parameter. Each model is constructed by defining a start model which is defined by at least one start value of the at least one topological parameter and the topological parameters are then fitted such that the model matches anatomical parameters within a predefined accuracy. The bone replacement element is then produced according to the model so obtained.

Currently, models for the production of bone replacements are used which are based on a limited number of simple geometric forms such as wave form, sine form, lattice structure, rhomb structure, honeycomb structure. Those forms are replicated, enlarged or scaled down. However, the microarchitecture of bones comprises different levels of hierarchy, which can be quantified by different morphometric bone parameters which depend on the localization within the skeleton as well as on properties of the patient as for example age and sex. The microarchitecture of a bone cannot be reproduced by simple geometric structures.

Currently it is not possible to scale biomimetic 3D CAD bone models such that the inner structure will be reproduced anatomically correct independently from the outer contour of the model. It is therefore currently not possible to respect the human bone microarchitecture with its different levels of hierarchy.

It is the object of the present invention to provide a method for producing a bone replacement element which allows a correct reproduction of different hierarchies and/or architectures of bone at a location where the bone replacement is to be inserted, and which at the same time allows scaling to the actual dimensions of the defect while maintaining the levels of hierarchy and/or architecture.

This object is solved by the method for producing a bone replacement according to claim 1 and the bone replacement element according to claim 16. The dependent claims describe advantageous embodiments of the method according to claim 1.

The present invention relates to a method for producing a bone replacement element. A bone replacement element can in particular be an element which is shaped to be inserted into a defect of an actual bone, for example in a human or an animal. It is desirable that the bone replacement element has an outer contour which matches the contour of the defect so that the bone replacement element fits within the defect without leaving a void between the bone replacement element and the bone and also reproducing the outer surface of the bone at the location of the defect.

According to the invention at least one image of a defect to be filled with the bone replacement is acquired. Preferably the at least one image is such that an outer contour, hull or envelope can be derived from the image. Advantageously the image can be a 3D image or a stack or series of 2D-images of the defect. For example, such an image can be acquired using a computed-tomography scan, CT, and/or a high resolution imaging with micro computed-tomography, µCT, in particular synchrotron based µCT. Advantageously, the contour can be determined using effective methods like standard CT, Magnetic resonance imaging (MRI), Cone Beam Computed Tomography (CBCT), Digital Volume tomography (DVT) or medical ultra sound with digital imaging and the µCT can be used to determine morphological (anatomical) parameters for different locations and/or cohorts. As the morphological parameters can be stored in a database, it can be sufficient to employ the effective methods for the image of the contour, hull or envelope. Thus, the image at the specific patient can be used to determine the architecture and the value ranges of the morphological (anatomical) parameters.

A stack or series of 2D-images can in particular be a plurality of images of the defect from different perspectives. Such a plurality of images can allow a reconstruction of the defect in three dimensions.

In a next step, an outer contour, hull or envelope of the defect is determined from the at least one image. The terms contour, hull and envelope shall be used synonymously within this document. The outer contour of the defect can for example be determined using image analysis. In an advantageous embodiment a CAD model can be generated of the outer contour. The outer contour can preferably be the global contour of the replacement element and/or the defect, which means that the outer contour can be the contour of the missing part in the bone which is to be filled with the bone replacement element.

The outer contour can for example be determined as follows from the at least one image. The outer contour of a defect can be constructed from independent variables (data points of the image) and a contour-model of the bone replacement element can be determined by the dependent variables that are to be fitted such that outer contour in the at least one image and the outer contour of the contour-model match each other to a desired accuracy.

The independent variables can first be acquired from the image and can optionally be manipulated to adequately represent the outer contour. The image can for example be seen as a pixel or point cloud. For identifying the outer contour in the image an "inside/outside" filter could be used, e.g. a grey scale filter. To represent the contour of the defect as a kind of surface representation that can identify the inside and outside regions, e.g. a polygon representation can be generated based on the pixel/point cloud acquired from the inside/outside filter. This can be done e.g. by a Delaunay triangulation algorithm. The vertexes of the triangles representing the outer contour would then be the independent variables. To increase or decrease the number of independent variables based on the image resolution a refinement triangulation scheme can be used.

The dependent variables of the contour-model likewise represent the outer contour of the model to be fitted and can be e.g. voxel parameter, the vertex coordinates of polygons representing the model, spline or shape parameters of NURBS, or control points of subdivision surfaces. For fitting the dependent variables of the bone replacement model or related start models to the independent ones, a fitting procedure can be employed. This can be done for example manually or automated.

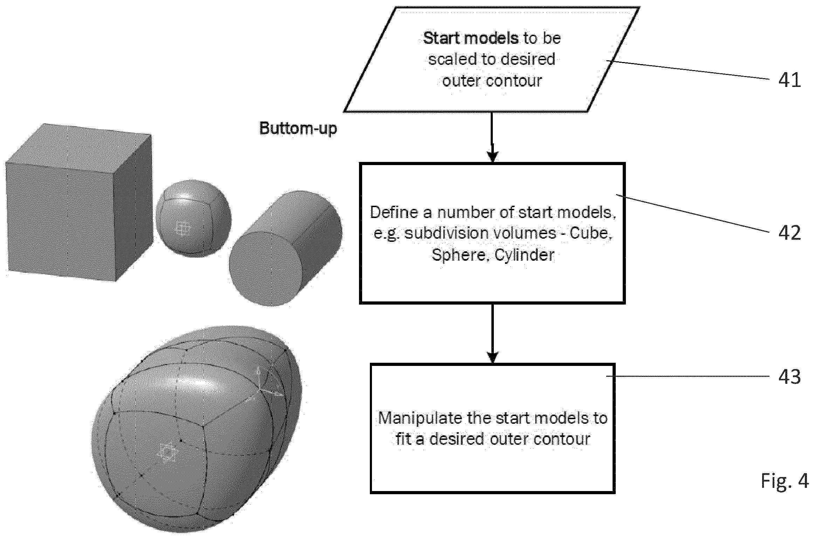

Manually this is possible through controlling of the dependent variables e.g. by manipulating the control points of a subdivision surface-based surface grid of the bone replacement element such that the outer contour of the bone replacement model adequately fits the set of data points representing the outer contour of the defect. For higher accuracy, the number of dependent variables can be enlarged to a finer mesh, e.g. the control point mesh for subdivision surfaces can be subdivided into a finer mesh of control points (e.g. Fig.4, reference sign 43).

Automated: this fitting procedure can also be automated. For this purpose, the outer contour of the defect can be represented again by a set of approximating data points, e.g. polygon vertexes. The dependent variables in the automated case could likewise represent the outer contour of the bone replacement (instead of related parameters of the geometry like the control points of subdivision surfaces or spline parameters). The fitting procedure can then be done by a regression algorithm e.g. the least squares method or minimum sum method to iteratively adapt the dependent variables to the desired degree of accuracy.

The regression algorithm can also be used, if the dependent variables are the related geometric parameters e.g. spline parameters or control points of subdivision surfaces. However, the accuracy of the fitting is still determined on the level of the outer contours and therefor an intermediate step creating a set of variables for the outer contour of the bone replacement model to be compared in the regression algorithm is recommended for better convergence.

According to the invention at least one region with a specific architecture is defined within the outer contour. Architectures can in particular be cortical bone architecture, spongious bone architecture, and/or trabecular architecture. Spongious and cortical bone are the most common architectures of bones. According to the invention it is determined which architectures shall be reproduced in which region of the bone replacement element. It is advantageous if the regions with specific architecture within the bone replacement element match the architectures of the bone material which are missing within the defect to be filled. That is, if for example the missing bone material in the contour of the defect contained a first region with a first architecture, e.g. spongious, as well as a second region with a second architecture, e.g. cortical architecture, it is advantageous to define within the outer contour determined in the at least one image a first region with the first architecture and a second region with the second architecture, wherein the regions have the same shape, dimensions and locations within the outer contour as the first and second regions have in the missing bone element.

According to the present invention at least one anatomical parameter is acquired of the at least one region. The term "anatomical parameter" here relates to parameters or properties of the actual bone which is missing in the defect. The anatomical parameters therefore define the anatomy of the bone. Different parameters can be used for different architectures. Examples for such kind of anatomical parameters can be porosity, bone volume fraction (relative density), bone surface fraction, trabeculae thickness, ratio of cortical to spongious.

In an advantageous embodiment the at least one anatomical parameter can be obtained from a database, in particular a database of human bone microarchitecture. Such a database can for example contain parameter profiles for different locations within the skeleton as well as different patient cohorts. Such a database can for example be generated by a survey of a large number of people or animals, for example using micro computed-tomography, µCT. Parameters which can be taken into account in order to choose a set of at least one anatomical parameter to be used for the bone replacement element can for example be age, sex, size, etc.

Alternatively, the anatomical parameter can also be obtained by measurement in the patient or animal having the bone defect to be filled by the bone replacement element. For example the anatomical parameters could be obtained from bone material surrounding the defect or from bone material at corresponding locations on the other side of the patient. If for example a defect is to be replaced in the left leg, anatomical parameters can be obtained from the corresponding position at the right leg.

According to the invention a model is constructed for each of the at least one regions with a specific architecture. This model will be defined by at least one topological parameter. Different kinds of models will be described below. The model can be understood in the sense of the mathematical model which is defined by parameters defining a topology or geometry. If for example the model is a mesh structure, such parameters could be angles and/or lengths of links between vertices. Many other kinds of models as for example non-uniform rational B-splines, NURBS, sets of geometric elements like tubes, cuboids, pyramids, cylinders, etc. are possible. The parameters defining these models can then be the topological parameters mentioned above.

According to the invention the construction of the model starts by defining a start model which is the model defined by at least one start value of the at least one topological parameter. Each topological parameter defining the model can be provided with a start value giving the model a specific starting shape.

Subsequently the topological parameters are fitted such that the model for each of the at least one different regions matches the anatomical parameters in the corresponding region with a predefined accuracy. The underlying idea is that the anatomical parameters which were chosen to characterize the defect, that is the bone material missing in the defect, can also be deduced from the model. If for example the porosity is used to define the bone material in the defect, the porosity will be derived from the model. Thus, in general if one or more anatomical parameters are used to define the bone material missing in the defect, the same parameters are derived from the model and compared with the anatomical parameters to define the missing material.

Changing the topological parameters of the model will also change the anatomical parameters derived from the model. The goal of the fitting is to change the topological parameters systematically until the anatomical parameters derived from the model match the anatomical parameters defining the bone material missing in the defect within a predefined accuracy.

The procedure for fitting topological parameters can for example be similar to the one for fitting of outer contours. The anatomical parameters derived from the image and matched with the database values can be the independent variables. The anatomical parameters derived from the model on basis of the topological parameters can be the dependent variables to be fitted. However, as the anatomical parameters of both the defect as well as the model should match, also the topological parameters can be seen as the dependent variables. Sometimes the topological parameters can be identical with the anatomical parameters but likely there is a (nonlinear) relationship between them. In any case, the topological parameters can be iteratively adapted such that the correlated anatomical parameters of the model match the anatomical parameters of the defect to the desired degree of accuracy.

Manually this could be done by parametric CAD construction e.g. manually setting the distances between vertexes, strut radii, thickness of a cortical region etc., and consecutive adaption, measuring and comparison. The process can optionally be made more efficient by approximating simple relations like the radii with the relative density of a representative region as design guidelines.

For automating the process, again, a least-squares estimation algorithm can be employed on the topological parameters and the anatomical parameters based on adequate start models and parametrization.

After the model has been fitted by altering the topological parameters so as to reproduce the anatomical parameters the model can be seen as being in a final state. According to the invention a bone replacement element is then produced according to the so obtained model, that is according to the model in the final state. In the production process the topology and/or geometry of the model can be produced with a suitable material.

In an advantageous embodiment of the invention at least one of the at least one topological parameters can define a local geometry of the corresponding model. For example the topological parameter could be the length of a specific link and/or an angle between two specific links meeting at a specific vertex. Using this kind of topological parameters defining a local geometry it is possible to define a model which can be directly fed into a 3D-printing process.

Advantageously at least one of the at least one anatomical parameters can define a density, a porosity, a gradient of density, and/or a gradient of a porosity of the bone. This kind of anatomical parameter is in particular useful where the architecture is spongious.

In an advantageous embodiment the model of at least one of the at least one regions can be constructed from nodes which are connected by links, wherein the topological parameters can comprise length of the links and/or radii or diameters of the links. Also parameters of an ellipse-equation and/or a rectangle, in particular with rounded corners, can be used for ellipse shaped or rectangle shaped cross sections. Latter shapes can also be used in a vertex and link structure, allowing the construction of e.g. plate-like structures, which often occur in trabeculae. Also angles can be used, although the length of the links can define the angles implicitly as well. Such kind of model would for example correspond to a mesh model.

In a preferred embodiment of the invention the model of at least one of the at least one regions can comprise vertex-based structures. Those kind of structures can for example be defined by spline parameterisation or Non-Uniform Rational B-splines, NURBS. Also the above mentioned mesh model can be regarded as vertex-based structure. The nodes are created based on vertices. The structure with NURBS can be constructed based on the vertex based mesh model.

An outline can be found in "Cellular Solids", 2nd edition, Lorna J. Gibson, Michael F. Ashby, University of Cambridge. Chapter 11 "Cancellous bone" (spongious bone) of this reference can be employed along the following remarks. The outer shell is a compact one (Cortical bone) enclosing a core of porous cellular, cancellous or trabecular bone. Trabecula means little beam in latin. Cancellous bone (spongious bone) is made up by a network of rods or plates. A network of rods produces low-density open cells, while plates give higher relative density. Any bone of relative density below 0.7 is usually classified as cancellous. The relative density can vary between 0.05 to 0.7

At the lowest densities the cells are open and like a network of rods. As the density increases, the rods progressively spread and flatten and become more plate-like and finally fuse to give almost closed cells.

In the context of the invention: The breakdown of the bone / bone replacement model can be done into two parts: (1) Cortical bone: the solid containing an interconnected network of void structures and (2) Spongious bone: a "cellular solid is one made up of an interconnected network of solid struts or plates which form the edges and faces of cells" (Quote from the textbook "cellular solids" (Gibson, Ashby), 2nd edit. Page 2). "From a geometrical point of view it is helpful to think of a cellular structure as vertices, joined by edges, which surround faces, which enclose cells" (Quote from the textbook "cellular solids" (Gibson, Ashby), 2nd edit. Page 30).

It is therefore justified to name both the interconnected network of void structures as well as the cellular structure/solid a vertex-based structure.

In a further advantageous embodiment of the invention the model of at least one of the at least one regions can be constructed from voxels. Voxels can in particular be volumes which together assemble the corresponding region. If for example the voxels are cubes, the topological parameters can be edge lengths and locations of the cubes as well as for example a flag which indicates whether a given voxel is filled with material or void.

Advantageously, the model of at least one of the at least one regions can define positive spaces, that is spaces which are filled with material. This kind of model is in particular advantageous for trabecular architecture where the model can define the trabeculae.

Advantageously, the model of at least one of the at least one regions can also define negative spaces, that is spaces within a volume of material which are void. This kind of model can advantageously be used for example for defining regions with spongious architecture. The model could for example define voids within the material. The model can also be used for cortical bone. In general the definition of negative spaces is advantageous for regions that have low porosity in comparison with high porosity areas like trabeculae.

The different kinds of models mentioned here can be used for both alternatives, that is for defining positive spaces as well as for defining negative spaces. If they define positive spaces they will define where the material is, if they define negative spaces they will define the voids within an otherwise homogeneous material.

In general, negative spaces can be used for cortical and/or for spongious architecture. Advantageously negative spaces can be employed above a certain relative density. For example negative spaces can be used for a relative density greater or equal 0,5 or greater or equal 0,7.

In an advantageous embodiment the start model for each of the at least one regions can be defined with an elementary shape as outer shape, as for example a cube, a sphere or a cylinder. In this embodiment, during or after the fitting the outer shape of each model is scaled such that all models combined completely fill the outer contour of the defect. This approach can be referred to as "bottom-up" approach. For this suitable shapes can be derived from the at least one image of the defect. For example one or more of said elementary shapes can be created and then be manipulated to fit the outer contour of the defect.

In an alternative approach, which can be referred to as "top down" approach, the volume defined by the outer contour can be divided into at least two regions, wherein for each region a start model is defined by defining a global volume in which the defect fully fits in, and then dividing the global volume into the at least two start models. The global volume can have any shape but should be so large that the complete contour of the defect can fit within the global volume without protruding beyond the global volume.

Each of the at least two start models can have a different architecture, that is for example spongious architecture, trabecular architecture, cancellous architecture, cortical architecture etc.

During the fitting of the topological parameters the regions can then assume an outer shape such that all regions together define the outer contour of the defect.

In an advantageous embodiment the bone replacement element can be produced according to the obtained model in a 3D printing method. In particular it is advantageous if the 3D printing method comprises selective laser melting, preferably using titanium as printing material.

Advantageously, a laser can be employed with a focus diameter which is about half of the smallest sections of the structures in the bone replacement to be printed. A layer thickness can preferably be equal or smaller than 20 µm.

Preferably, a laser power can be used, which is lower than usually used for bulk material to achieve a penetration depth in the range of the layer thickness (e.g. smaller or equal 20 µm).

Advantageously, a soft coating device (e.g. polymer) can be used for homogenous distribution of feedstock and less interference during coating with already printed structures.

In the following the invention shall be described by way of examples with reference to the Figures. Same reference signs denote same or corresponding features. The features described in the context of the examples can also be realized independently from the examples and can be combined between different examples.
Figure 1 shows an example of the method according to the invention,
Figure 2 shows an example of a process in which a model can be defined,
Figure 3 shows in detail how the type of model can be decided,
Figure 4 shows an example how a start model for one of the regions with a specific architecture can be defined,
Figure 5 shows an example for a possible model which can be used for a specific region,
Figure 6 shows two examples of possible positive start models,
Figure 7 shows two different structures which can be constructed in a way as shown in Figure 5, and
Figure 8 shows an example how models as created in Figures 5 and 7 can be combined with start models as shown in Figure 6.

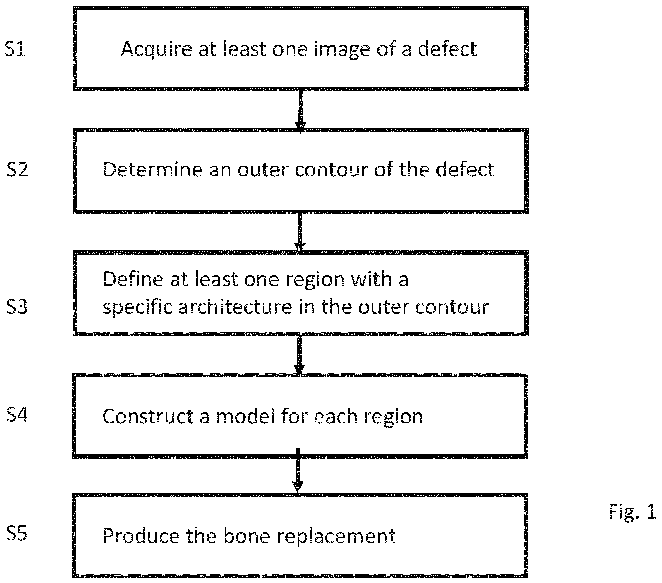

Figure 1 shows a general flow chart of an example of the method for producing a bone replacement element according to the present invention. In a first step S1 at least one image of a defect to be filled with the bone replacement is acquired. In a second step S2 an outer contour of the defect is determined from the at least one image. In a further step S3 at least one region with a specific architecture is defined within the outer contour. In a further step S4 a model is constructed for each region and in another step S5 the bone replacement is produced according to the obtained model.

The model constructed in step S4 is defined by at least one topological parameter. To construct the models a start model is defined for each model. The start models are defined by at least one start value of the at least one topological parameter. The topological parameters are subsequently fitted such that the model of each of the different regions matches anatomical parameters in the corresponding region with a predefined accuracy. The anatomical parameters can for example be obtained from a database. They define properties of the bone which is missing in the region of the defect. Anatomical parameters can for example be a density, a porosity, a gradient of a density and/or a gradient of a porosity of the bone at the location of the corresponding region. Possible architectures can for example be cortical bone and/or spongious with/without trabecular structures. A low porosity for example can represent fine negative channel structures which can be synchronized to a desired mean porosity. A high porosity can for example relate to negative channel structures, for example with more nodes, connections or at some threshold porosity, adding another layer of inner channel structure. A trabecular structure can for example be created by positive channels that match sizing factors of the anatomical parameters defining trabecular data.

Gradient information can be reproduced by combining different low and high porosity volumes whereby channel connections are advantageously matched, for example with matching outer nodes to remain an open-structure. Gradient information can also be reproduced by gradients in channel nodes and sizing of channels.

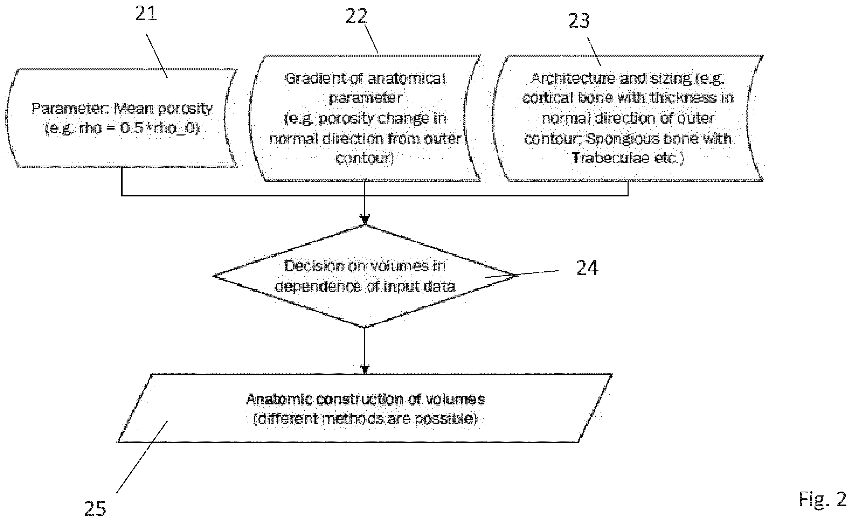

Figure 2 shows an example of a process in which a model can be defined. Different anatomical parameters can be obtained from the bone which is missing in the defect. For example a mean porosity as shown in 21, gradient information, for example a porosity change in a normal direction from the outer contour as shown in 22 as well an architecture and sizing (for example cortical bone with thickness in normal direction of outer contour, spongious b one with trabeculae etc.) as shown in 23 can enter the decision on the architecture in 24. Based on this decision regions with a specific architecture can be defined in 25.

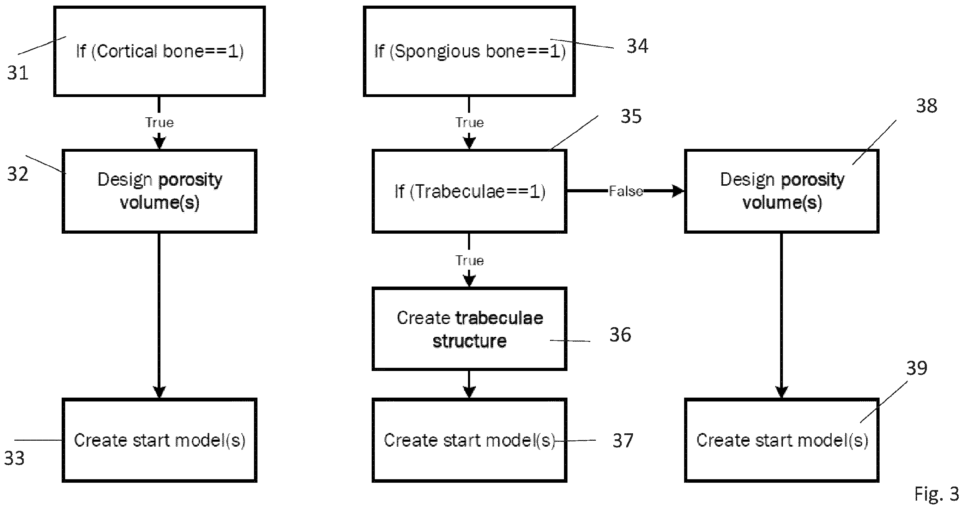

Figure 3 shows in detail an example how the type of model can be decided. If in a specific region of the defect the bone used to be cortical bone 31 porosity volumes can be designed 32 and start models can be created as porosity volumes 33.

If the bone material at the specific location is spongious 34 it can furthermore be decided whether the architecture is trabecular or not in 35. If the architecture is trabecular a trabeculae structure can be created as model 36 and start models can be created with trabecular architecture 37. If on the other hand the architecture is not trabecular, porosity volumes can be designed 38 and start models with porosity design can be created 39.

Figure 4 shows an example how a start model for one of the regions with a specific architecture can be defined and then scaled such that it fills a certain volume of the outer contour. Assume there is a start model which is to be scaled to a desired outer contour in 41. First a number of start models, for example subdivision volumes of the outer contour, are defined in 42, for example cubes, spheres or cylinders. The start models are then manipulated in 43 to fit the desired outer contour.

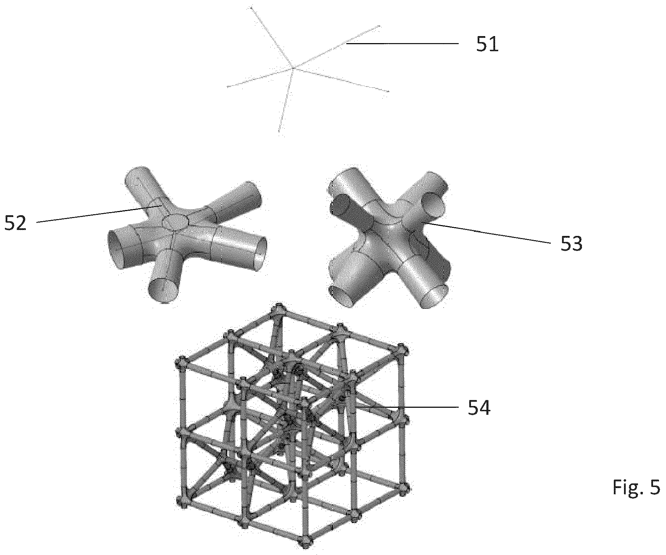

Figure 5 shows an example for a possible model which can be used for a specific region. The model is in general based on links which are connected via nodes as shown with reference sign 51. 51 defines a vertex for the links. Topological parameters of such kind of model could for example be the length of the links. Optionally also the angles between the links can be used as topological parameters. In order to reproduce a thickness of the structures in the model the links can be assigned a radius so that a cylindrical structure surrounds each of the links, the link being the cylinder axis. Also parameters of an ellipse equation and/or a rectangle with rounded corners can be used a alternative cross section shapes. These can also be based on said vertex and link structure, which also allows the creation of e.g. plate like structures, often occurring in trabeculae. Such kind of models are shown with reference signs 52 and 53. The radii can be additional topological parameters. Using elements 51, 52 and/or 53 a model 54 can be constructed for a specific region.

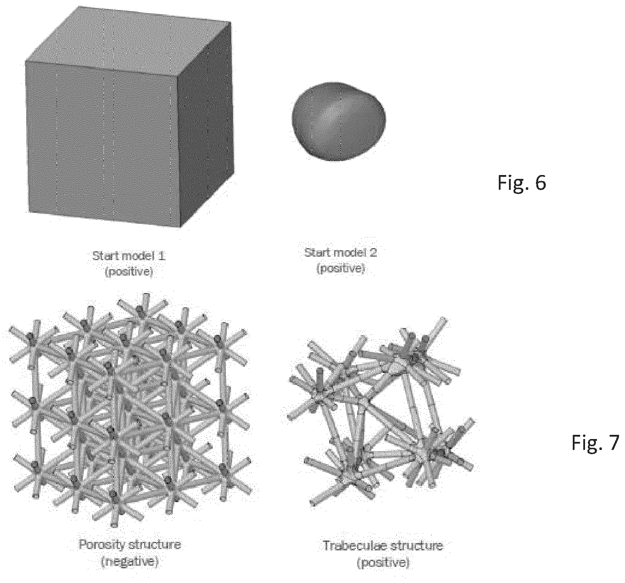

Figure 6 shows two examples of possible positive start models, the left one being a cube, the right one being a more complex structure. These start models are positive which means that they are assumed to be fully filled with material.

Figure 7 shows two different structures which can be constructed in a way as shown in Figure 5. These structures can be used as positive or as negative structures. If these structures are used as positive structures the elements visible in Figure 7 are filled with material. These kind of structures can be directly printed in a 3D-printing process where the shown elements are provided with material. If these structures are used as negative structures the elements visible in Figure 7 can define the voids within an otherwise full block of material.

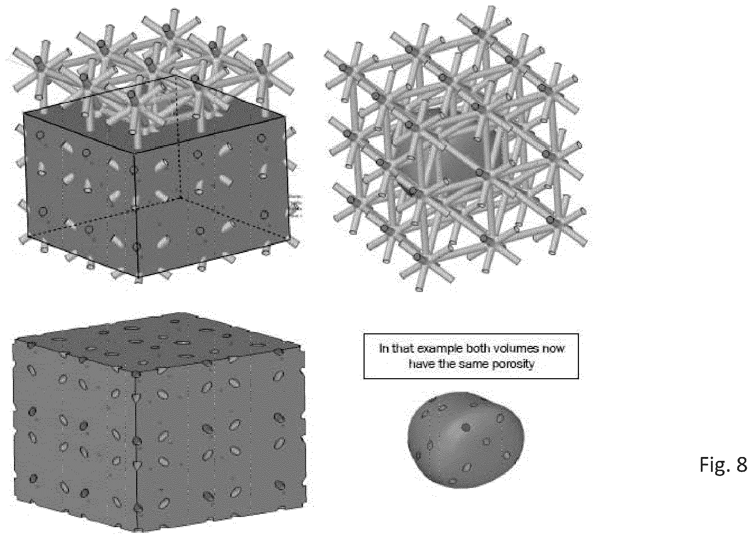

Figure 8 shows an example how models as created in Figures 5 and 7 can be combined with start models as shown in Figure 6 in order to create negative structures. To achieve a negative structure the model shown in Figure 7 is superimposed over the start model. The model then defines those regions where no material is present. Mathematically spoken the model is deducted from the start model. This kind of construction is in particular helpful for constructing porous structures.

## Claims

1. Method for producing a bone replacement element, the method comprising the following steps:
acquiring at least one **image** of a defect to be filled with the bone replacement,
determining an **outer contour** of the defect from the at least one image,
defining at least one **region** with a specific **architecture** within the outer contour,
acquiring at least one **anatomical parameter** of the at least one region, constructing a **model** for each of the at least one regions, which model is defined by at least one **topological parameter,**
wherein each model is constructed by defining a **start model** which is the model defined by at least one start value of the at least one topological parameter, and
subsequently **fitting** the **topological parameters** such that the **model** for each of the at least one different regions matches the **anatomical parameters** in the corresponding region within a predefined accuracy, **producing** the **bone replacement** element according to the so obtained model.

2. Method according to the preceding claim, wherein at least one of the at least one topological parameters defines a **local geometry** of the corresponding model.

3. Method according to any of the preceding claims, wherein at least one of the at least one anatomical parameters defines a **density, a porosity,** a **gradient** of a density and/or a gradient of a porosity of the bone.

4. Method according to one of the preceding claims, wherein the model of at least one of the at least one regions comprises **nodes** connected by **links,** wherein the topological parameters comprise **lengths** of the links and radii or **diameters** of the links.

5. Method according to any of the preceding claims, wherein the model of at least one of the at least one regions comprises vertex-based structures, which are preferably defined by **spline parametrization** or Non-Uniform Rational B-Splines, NURBS.

6. Method according to one of the preceding claims wherein the model of at least one of the at least one regions is constructed from **voxels.**

7. Method according to any of the preceding claims, wherein
the specific architecture of at least one of the at least one regions is one of **spongious** architecture or trabecular **architecture or cortical architecture.**

8. Method according to any of the preceding claims, wherein the model of at least one of the at least one regions, a relative density of which is preferably smaller or equal 0.5, defines **positive spaces** which are filled with material, and/or the model of at least one of the at least one regions, a relative density of which is preferably greater than 0.5, more preferably greater than 0.6, defines **negative spaces** within a volume of material which are void.

9. Method according to any of the preceding claims, wherein the start model for each of the at least one regions is defined with an elementary shape as outer shape, preferably a **cube,** a **sphere** or a **cylinder,** and wherein during or after the fitting the outer shape of each model is **scaled** such that all models combined **completely fill** the outer contour of the defect.

10. Method according to any of claims 1 to 8, wherein at least two start models are defined by defining a **global volume** in which the defect fully fits in, and
**dividing** the global volume into the at least two start models.

11. Method according to one of the preceding claims, wherein the at least one image is a 3D-image or a plurality of images of the defect from different directions.

12. Method according to one of the preceding claims, wherein the at least one image is generated in a Computed Tomography Scan, Magnetic resonance imaging ,MRI, Cone Beam Computed Tomography ,CBCT, Digital Volume tomography ,DVT, medical ultra sound with digital imaging and/or a Micro-Computed-Tomography scan.

13. Method according to one of the preceding claims, wherein the at least one anatomical parameter of the at least one region is retrieved from a database.

14. Method according to one of the preceding claims, wherein the producing of the bone replacement element is done in a 3D printing method.

15. Method according to the preceding claim, where in the 3D printing method comprises selective laser melting, preferably using titanium as a printing material.

16. Bone replacement element, **characterized in that** the bone replacement element is produced in a method according to any of the preceding claims.
